Europäisches Patentamt

European Patent Office

Office européen des brevets

(11) Publication number: **0 044 510**
**B1**

## (12) EUROPEAN PATENT SPECIFICATION

(45) Date of publication of patent specification: **07.03.84**

(21) Application number: **81105520.1**

(22) Date of filing: **14.07.81**

(51) Int. Cl.$^3$: **C 07 C  69/80,**
**C 07 C  69/82,**
**C 07 C  67/08, C 07 C  67/10**
**//B01J23/14**

(54) Process for the preparation of diaryl esters.

(30) Priority: **18.07.80 US 170995**

(43) Date of publication of application:
**27.01.82 Bulletin 82/4**

(45) Publication of the grant of the patent:
**07.03.84 Bulletin 84/10**

(84) Designated Contracting States:
**DE FR GB IT NL**

(56) References cited:
**GB - A - 999 947**
**US - A - 3 694 490**
**US - A - 3 705 186**
**US - A - 3 772 389**

(73) Proprietor: **GENERAL ELECTRIC COMPANY**
**1 River Road**
**Schenectady New York 12305 (US)**

(72) Inventor: **Fox, Daniel Wayne**
**193 Dawes Avenue**
**Pittsfield, Mass. 01201 (US)**
Inventor: **Shafer, Sheldon Jay**
**40 Day Street**
**Pittsfield, Mass. 01201 (US)**
Inventor: **Kaduk, Bruce Alexander**
**57 Easton Avenue**
**Pittsfield, Mass. 01201 (US)**

(74) Representative: **Schüler, Horst, Dr. et al,**
**Kaiserstrasse 41**
**D-6000 Frankfurt/Main 1 (DE)**

Courier Press, Leamington Spa, England.

Process for the preparation of diaryl esters

## Background of the invention

Various methods have been disclosed for preparing diaryl esters of aromatic carboxylic acids, such as isophthalic and terephthalic acids, by reacting the acids with an aromatic monohydroxy compound, for example, phenol. In this connection reference is made to U.S. Letters Patents Nos. 3,413,336 granted November 26, 1968 and 4,124,566 granted November 7, 1978.

In U.S. Patent 3,413,336 an additional agent, acetic anhydride, is employed in carrying out the reaction which is used "with equivalent amounts or a slight excess of a phenol" (col. 2, lines 31—32). In col. 3, lines 2—3, it is also stated: "it is generally not advisable for reasons of economics, to use an excess of more than 25%". The specific Examples I—IV disclose respectively "2 moles+25%, "2 moles+10%", "2 moles+20%" and "2 moles+20%".

In U.S. Patent No. 4,124,566 it is stated that in "...the most preferred state of the esterification reaction...the aromatic monohydroxy compound (phenol) is present only in a low concentration in the aromatic dicarboxylic acid slurry". (col. 6, lines 29—34). In the Examples, the molar ratio of the phenol to the acid is about 1 to 4 and 1 to 6 moles. Furthermore, an aromatic hydrocarbon medium such as ethylbenzene is required in the process.

U.S. Patent 3,694,490 describes the use of a large molar excess of the aromatic hydroxy compound which serves both as a reactant and a solvent. The reaction takes place in a closed system (an autoclave) at a temperature of above 275°C. After cooling down the precipitated unreacted acid is filtered out, and the filtrate containing the desired ester, unreacted phenol and the water by-product is separated by distillation.

G.B. Patent 999 947 describes the use of stannous oxide as catalyst in a reaction for preparing carboxylate esters.

## Summary of the invention

It has now been found that the diaryl esters of aromatic dicarboxylic acids can be readily, simply and in a much better yield prepared by a process which comprises reacting an aromatic dicarboxylic acid with an aromatic monohydroxy compound in which said acid is soluble and with which it forms a solution, said reaction being carried out by heating said solution at an elevated temperature to form the diaryl ester and removing the water formed during the reaction, which is characterized by the use of an excess of said aromatic monohydroxy compound serving as a carrier to remove the water and resulting in a product of 90—95% of the diaryl ester with a content of 5—10% of the corresponding monoaryl ester of the dicarboxylic acid.

The large excess of the aromatic monohydroxy compound can be, for example, in a molar ratio of 15 to 17 moles to one mole of the dicarboxylic acid.

The 15—17 moles of aromatic hydroxy compound specified is a convenient but not necessary excess. One may accomplish the same results for example by initially using one half this quantity and adding the remainder at temperature under pressure after aromatic hydroxy compound along with water of reaction has been distilled. Still another option would be to distill the aromatic hydroxy compound and water of reaction under pressure, remove the water, recycle the aromatic hydroxy compound without cooling or depressurizing. The subject process has the advantage among others, of eliminating the use of acetic anhydride, and aromatic hydrocarbons, such as ethylbenzene, heretofore employed. The excess aromatic monohydroxy compound, such as phenol, which is employed is readily recovered and can be reused by returning it to a new reaction batch.

In order to obtain diaryl ester with a total yield of 99.9% or substantially pure diaryl ester the resulting reaction product is heated with a stoichiometric excess of a diaryl carbonate to completely convert the monoaryl ester content thereof.

## Description of the preferred embodiment

In general the process of the subject invention comprises reacting an aromatic dicarboxylic acid with a large molar excess of an aromatic monohydroxy compound which reacts with said acid, and in which said acid is soluble, and with which it forms a solution. The reaction is carried out by heating said solution at an elevated temperature to form the diaryl ester, and removing water formed during the reaction and a major portion of the unreacted aromatic monohydroxy compound. The aromatic monohydroxy compound serves as a carrier to remove the water, said reaction resulting in a mixture of 90—95% of the diaryl ester and 5—10% of the corresponding monoaryl ester of the dicarboxylic acid.

As aromatic dicarboxylic acids it is preferred to use a member of the group consisting of isophthalic and terephthalic acid, and their derivatives such as 5-chloroisophthalic or methyl-terephthalic or dichloroterephthalic acid.

As an example of the aromatic monohydroxy compounds it is preferred to use phenol. Other examples are isomeric cresols, or xylenols, butyl phenol, benzyl phenol, m-cresol, and $\beta$-naphthol, and the like.

The aromatic monohydroxy compound is employed in a molar ratio of 1 mole of the aromatic dicarboxylic acid to 15—17 moles of the aromatic monohydroxy compound.

The heating and distillative removal of by-product water and aromatic hydroxy compound is carried out at an elevated temperature of 275°C to 300°C. for 1 hour to 5 hours, preferably 3 to 5 hours. This generally suffices to remove 90—95% of the water of reaction which translates into a 90—95% yield of diester.

It is preferred to carry out the reaction in the presence of stannous oxide (SnO) as a catalyst, in an amount of 0.1 to 0.5 mole percent based on the aromatic dicarboxylic acid. The resulting product comprises 90—95% of the diaryl ester of the aromatic dicarboxylic acid plus a content of 5—10% of the corresponding monoaryl, or half ester, of the aromatic dicarboxylic acid. If a pure diaryl ester is desired, purification of the afore-said product is carried out by reacting it with a stoichiometric excess of a diaryl carbonate in a molar ratio of 1.5 mole to 4 moles of the diaryl carbonate to one mole of the half ester content in the crude product to completely convert the monoaryl ester to the diaryl ester. The reaction is carried out at a temperature of from 245°C to 300°C for 30 minutes to 2 hours.

The reaction mixture is then distilled at a gradually higher vacuum and temperature with aromatic monohydroxy compound being removed first, then the excess diaryl carbonate, and finally pure diaryl ester of the aromatic dicarboxylic acid with a yield of 99.9%.

Thus, the subject invention also provides a process particularly advantageous for the purification of diphenyl isophthalate and diphenyl terephthalate where these esters are prepared by reacting phenol with isophthalic or terephthalic acid. In the reaction of these acids with phenol some corresponding monophenyl or half ester, of isophthalic or terephthalic acid is formed and remains in the resulting product as an impurity. The half ester cannot be removed by distillation.

In general, the subject process for removing the undesired half ester to obtain the pure diester comprises reacting a stoichiometric excess of diphenyl carbonate in a molar ratio of 1.5 moles to 4 moles of the diphenyl carbonate to 1 mol of the half ester content in the crude product at an elevated temperature of 245°C to 300°C for 30 minutes to several hours, for example, 2 hours, to completely convert the half ester into the diester, and then distilling the reaction product to obtain pure diphenyl isophthalate or diphenyl terephthalate.

Neither of the above mentioned U.S. Patent Nos. 3,413,336 or 4,124,566 refer to or deal with the half ester impurity or its conversion to the diester. In U.S. Patent 3,413,336, column 3, lines 39—42 it is stated: "The diaryl esters are obtained in a yield of over 90%. They may be easily obtained in any desired degree of purity by simple distillation or recrystallizatin". In Examples I—IV the diaryl ester is distilled off to give yields of 93%, 90%, and 90.5%, respectively.

In U.S. Patent No. 4,124,566, which is concerned with preparing polyesters, the diaryl ester product obtained in the first step is directly converted into polymer by reaction with an aromatic dihydroxy compound, such as bisphenol A. There is no mention of a half ester or purification of the diaryl ester product either by crystallization or distillation before it is converted into the polymer.

The subject invention is further described in the Examples which follow. These examples are preferred embodiments and are not to be construed as limitations.

Example I

Terephthalic acid (1 mole), phenol (15 moles) and stannous oxide (SnO) as catalyst (0.1—0.5) mole% based on the terephthalic acid) are charged into an autoclave, provided with a mechanical stirrer and a needle valve to which is connected a distillation column. The reaction mixture is heated to 300°C over the course of 1.5 hours during which time the pressure rises to 17,5 bar. The needle valve is opened and phenol and water formed during the reactions are distilled over the course of three hours. During this period 60% of the original amount of phenol is distilled off. Phenol is used in this reaction, as a reagent, as a solvent for the acid, and as a carrier to remove the water. The initial distilling phenol contains 14% by weight of water while the final distilling phenol contains about 0.2% water.

A product is obtained which contains approximately 95% of diphenyl ester and approximately 5% of monophenyl ester. The total yield of diphenyl ester, if desired, can be increased and a purer product obtained by treating the mixture with diphenyl carbonate (molar ratio of 1.5 moles of diphenyl carbonate to 1 mole of the monophenyl ester) and heating to 300°C for about 30 minutes after which time the 5% monophenyl ester content is completely converted to diphenyl terephthalate to give a total yield of 99.9% of said diphenyl terephthalate.

The reaction mixture is then distilled with phenol removed at 200—220°C/4000—1300 Pa diphenyl carbonate excess removed at 240—260°C/1330—2660 Pa and, finally, the diphenyl terephthalate recovered at 240—260°C/133 Pa.

Example 2

Example 1 is repeated except that the same amount of isophthalic acid is employed. The initial product obtained contains approximately 95% of diphenyl isophthalate and approximately 5% of the monophenyl ester of isophthalic acid. When it is further treated as described in the second and third paragraphs of Example 1, a total yield of diphenyl isophthalate of 99.9% is obtained.

The following Examples which follow are preferred embodiments of the novel process of the invention for purifying products comprising diaryl esters of aromatic dicarboxylic acid and the corresponding monoaryl, or half ester, of aromatic carboxylic acids.

Example 3

Terephthalic acid (1 mole), phenol (10 moles) and stannous oxide as catalyst (0.1—0.5 mole % based on the terephthalic acid) are charged into an autoclave provided with a mechanical stirrer and a needle valve to which is connected a distillation column. The reaction mixture is heated to 300°C over the course of 1.5 hours during which time the pressure rises to 17,5 bar. The needle valve is opened and phenol and water formed during the reaction are distilled off. At the end of 1 hour of distillation, an additional 2.5 moles of phenol is added to the autoclave under pressure. This is repeated at the end of 2 hours of distillation. Over a period of 3 hours 60% of the total quantity of phenol charged to the autoclave is distilled off. Phenol is used in this reaction, as a reagent, as a solvent for the acid, and as a carrier to remove the water. The initial distilling phenol contains 14% by weight of water while the final distilling phenol contains about 0.2% water.

A product is obtained which contains approximately 95% of diphenyl ester and approximately 5% of monophenyl ester. The total yield of diphenyl ester, if desired, can be increased as outlined in Example 1.

Example 4

Example 3 is repeated except that the same amount of isophthalic acid is employed. The initial product obtained contains approximately 5% of the monophenyl ester of isophthalic acid. When it is further treated as described in the second and third paragraphs of Example 1 a total yield of diphenyl isophthalate of 99.9% is obtained.

Example 5

Crude diphenyl terephthalate (50 g) obtained by the process described in Examples 1 and 2 and containing approximately 60.9% phenol, 6.7% monophenyl terephthalate (half acid ester, and 32.4% diphenyl terephthalate is charged to a 250 ml, 3-necked flask equipped with a mechanical stirrer, Vigreux column and nitrogen (N₂) inlet. At this time, 9.0 grams of diphenyl carbonate (3:1 molar ratio based on the half acid ester) and 0.10 grams of stannous oxide (0.5 mole % based on the half acid ester) are added to the reaction flask and the mixture is heated to a pot temperature of 280—290°C for approximately 2 hours. This procedure results in conversion of the half-acid ester to diphenyl terephthalate. The mixture is then distilled with phenol removed at 200—220°C/4000—13000 Pa diphenyl carbonate excess if removed at 240—260°C/1300—2600 Pa, and finally, diphenyl terephthalate is recovered at 240—260°C/133 Pa.

Example 6

Crude diphenyl isophthalate (50 g) obtained by the process described in Examples 1 and 2 and containing approximately 55.2% phenol, 2.8% monophenyl isophthalate (half acid ester), and 42.0% diphenyl isophthalate is charged to a 250 ml flask as in Example 5. At this time, 3.7 grams of diphenyl carbonate (3:1 molar ratio based on half acid ester) and 0.05 grams of stannous oxide (0.50 mole % based on the half acid ester) are added to the reaction flask and the mixture is heated to a pot temperature of 280—290°C for approximately 2 hours, resulting in conversion of the half acid ester to diphenyl isophthalate. The mixture is then distilled as in Example 5 with diphenyl isophthalate recovered at 240—260°C/133 Pa.

The subject invention also embraces the process of preparing diaryl esters of aromatic dicarboxylic acids by reacting a monaryl ester of a dicarboxylic acid, that is the half ester, as such, not as an impurity in a crude diaryl ester of a dicarboxylic acid with a diaryl carbonate. In general the subject process comprises reacting a stoichiometric excess of a diaryl carbonate (from about 1.5 to about 4 moles based on the amount of half-ester) at an elevated temperature of about 250°C to about 300°C for about 30 minutes to two hours to completely convert the half ester into the diaryl ester, and recovering the latter, as for example, by distillation.

When the process is applied to reacting with diphenyl carbonate monophenyl terephthalate or monophenyl isophthalate (half-esters), the proportion of reagents and conditions are the same as set forth in Examples 3 to 6, except that the half-esters, as such, are employed as the starting materials instead of crude diphenyl terephthalate or crude diphenyl isophthalate in which the half esters are present as an impurity.

The foregoing detailed description and examples will suggest many variations to those skilled in the art. All such variations are within the full scope of the appended claims.

**Claims**

1. A process for preparing diaryl esters of aromatic diarboxylic acids which comprises reacting an aromatic dicarboxylic acid with an aromatic monohydroxy compound, in which said acid is soluble and with which it forms a solution, said reaction being carried out by heating said solution at an elevated temperature to form the diaryl ester, and removing the water formed during the reaction, characterized by the use of an excess of said aromatic monohydroxy compound serving as a carrier to remove the water, and resulting in a product of 90—95% of the diaryl ester with a content of 5—10% of the corresponding monoaryl ester of the dicarboxylic acid.

2. A process according to claim 1, wherein the resulting product is heated with a stoichiometric excess of a diaryl carbonate to completely convert the monoaryl ester content thereof to the diaryl ester to obtain substantially pure diaryl ester with a total yield of 99.9%.

3. A process according to claim 1, wherein the aromatic dicarboxylic acid is a member of the group consisting of isophthalic acid and terephthalic acid.

4. A process according to claim 1, wherein the aromatic dicarboxylic acid is isophthalic acid.

5. A process according to claim 1, wherein the aromatic dicarboxylic acid is terephthalic acid.

6. A process according to claim 1, wherein the aromatic dicarboxylic acid is a member of the group consistinjg of isophthalic acid and terephthalic acid, and the aromatic monohydroxy compound is phenol.

7. A process according to claim 1, wherein the molar ratio of the aromatic dicarboxylic acid to the aromatic monohydroxy compound is 1 to 15—17, the heating is carried out at 275°C to 300°C for 5 hours, 60—70% of the aromatic monohydroxy compound is removed from the reaction mixture along with the water formed during the reaction by distillation over a period of 3—5 hours and a product comprising 90—95% of the diaryl ester with a content of 5—10% of the monoaryl ester is obtained.

8. A process according to claim 1, wherein stannous oxide is employed as a catalyst in the reaction.

9. A process according to claim 7, wherein stannous oxide is employed as a catalyst and the resulting product obtained is heated with a stoichiometric excess of a diaryl carbonate to completely convert the monoaryl ester to obtain purified diaryl ester.

10. A process according to claim 9, wherein the aromatic dicarboxylic acid is a member of the group consisting of isophthalic and terephthalic acid, the aromatic hydroxy compound is phenol and the diaryl carbonate is diphenyl carbonate, and the diaryl ester obtained is a member of the group consisting of diphenyl isophthalate and diphenyl terephthalate.

11. A process for purifying a crude diaryl ester of an aromatic dicarboxylic acid having present as an impurity the corresponding monoaryl half ester of such acid, which comprises reacting said crude product with a stoichiometric excess of a diaryl carbonate at a temperature of 245°C to 300°C for from 30 minutes to 2 hours to completely convert the half ester to the diaryl ester, and then distilling the reaction product to recover pure diaryl ester of the aromatic dicarboxylic acid.

12. A process according to claim 11, in which the diaryl ester is a member of the group consisting of diphenyl isophthalate and diphenyl terephthalate and the monoaryl half ester is a member of the group consisting of monophenyl isophthalate and monophenyl terephthalate.

13. A process according to claim 11 wherein the reaction is carried out in the presence of stannous oxide as the catalyst.

14. A process according to claim 12 wherein the diaryl ester is diphenyl isophthalate and the monoaryl half ester is monophenyl isophthalate.

15. A process according to claim 12 wherein the diaryl ester is diphenyl terephthalate and the monoaryl half ester is monophenyl terephthalate.

16. A process which comprises reacting a monoaryl ester of an aromatic dicarboxylic acid with a stoichiometric excess of a diaryl carbonate at an elevated temperature to produce the corresponding diaryl ester of the aromatic dicarboxylic acid and recovering the diaryl ester.

17. A process according to claim 16, wherein the monoaryl ester is a member of the group consisting of monophenyl isophalate and monophenyl terephthalate and the diaryl carbonate is diphenyl carbonate.

18. A process according to claim 16 wherein the molar ratio of the diaryl carbonate to the monoaryl ester is 1.5 moles to 4 moles of the former to 1 mole of the latter.

19. A process according to claim 18, wherein the reaction is carried out at a temperature of 250°C to 300°C and maintained for 30 minutes to 2 hours.

## Patentansprüche

1. Verfahren zur Herstellung von Diarylestern aromatischer Dicarbonsäuren, bei dem eine aromatische Dicarbonsäure mit einer aromatischen Monohydroxyverbindung umgesetzt wird, in der die Säure löslich ist und mit der sie eine Lösung bildet, wobei die Reaktion unter Erhitzen der Lösung auf eine erhöhte Temperatur unter Bildung des Diarylesters und Entfernung des während der Reaktion gebildeten Wassers durchgeführt wird, gekennzeichnet, durch die Verwendung eines Überschusses der aromatischen Monohydroxyverbindung als Träger zur Entfernung des Wassers, wobei ein Produkt aus 90 bis 95% des Diarylesters mit einem Gehalt von 5 bis 10% des entsprechenden Monoarylesters der Dicarbonsäure erhalten wird.

2. Verfahren nach Anspruch 1, worin das erhaltene Produkt mit einem stöchiometrischen Überschuß eines Diarylcarbonats zur vollständigen Umwandlung des Monoarylestergehaltes desselben in den Diarylester erhitzt wird, um im wesentlichen reinen Diarylester mit einer Gesamtausbeute von 99,9% zu erhalten.

3. Verfahren nach Anspruch 1, worin die aromatische Dicarbonsäure ein Glied aus der Gruppe bestehend aus Isophthalsäure und Terephthalsäure ist.

4. Verfahren nach Anspruch 1, worin die aromatische Dicarbonsäure Isophthalsäure ist.

5. Verfahren nach Anspruch 1, worin die aromatische Dicarbonsäure Terephthalsäure ist.

6. Verfahren nach Anspruch 1, worin die aromatische Dicarbonsäure ein Glied aus der Gruppe bestehend aus Isophthalsäure und Terephthalsäure, und die aromatische Monohydroxyverbindung Phenol ist.

7. Verfahren nach Anspruch 1, worin das molare Verhältnis der aromatischen Dicarbonsäure zur aromatischen Monohydroxyverbindung 1 bis 15—17 ist, das Erhitzen 5 Std. lang auf 275 bis 300°C durchgeführt wird, 60 bis 70% der aromatischen Monohydroxyverbindung zusammen mit dem während der Reaktion gebildeten Wasser durch Destillation innerhalb von 3 bis 5 Std. aus der Reaktionsmischung entfernt wird und ein 90 bis 95% des Diarylesters enthaltendes Produkt mit einem Gehalt von 5 bis 10% des Monoarylesters erhalten wird.

8. Verfahren nach Anspruch 1, worin Zinn(II)-oxid in der Reaktion als Katalysator verwendet wird.

9. Verfahren nach Anspruch 7, worin Zinn(II)-oxid als Katalysator verwendet wird und das erhaltene Produkt mit einem stöchiometrischen Überschuß eines Diarylcarbonats erhitzt wird, um den Monoarylester vollständig umzuwandeln und gereinigten Diarylester zu erhalten.

10. Verfahren nach Anspruch 9, worin die aromatische Dicarbonsäure ein Glied aus der Gruppe bestehend aus Isophthalsäure und Terephthalsäure ist, die aromatische Hydroxyverbindung Phenol und das Diarylcarbonat Diphenylcarbonat und der erhaltene Diarylester ein Glied aus der Gruppe bestehend aus Diphenylisophthalat und Diphenylterephthalat ist.

11. Verfahren zur Reinigung eines rohen Diarylesters einer aromatischen Dicarbonsäure, die als Verunreinigung den entsprechenden Monoarylhalbester dieser Säure enthält, welches die 30 Min. bis 2 Std. dauernde Umwandlung des rohen Produktes mit einem stöchiometrischen Überschuß eines Diarylcarbonats bei einer Temperatur von 245 bis 300°C zur vollständigen Umwandlung des Halbesters in den Diarylester und dann die Destillation des Reaktionsproduktes zur Gewinnung des reinen Diarylesters der aromatischen Dicarbonsäure umfaßt.

12. Verfahren nach Anspruch 11, bei dem der Diarylester ein Glied ist aus der Gruppe bestehend aus Diphenylisophthalat und Diphenylterephthalat und der Monoarylhalbester ein Glied ist aus der Gruppe bestehend aus Monophenylisophthalat und Monophenylterephthalat.

13. Verfahren nach Anspruch 11, worin die Reaktion in Anwesenheit von Zinn(II)-oxid als Katalysator durchgeführt wird.

14. Verfahren nach Anspruch 12, worin der Diarylester Diphenylisophthalat und der Monoarylhalbester Monophenylisophthalat ist.

15. Verfahren nach Anspruch 12, worin der Diarylester Diphenylterephthalat und der Monoarylhalbester Monophenylterephthalat ist.

16. Verfahren, welches die Umsetzung eines Monoarylesters einer aromatischen Dicarbonsäure mit einem stöchiometrischen Überschuß eines Diarylcarbonats bei einer erhöhten Temperatur unter Bildung des entsprechenden Diarylesters der aromatischen Dicarbonsäure und die Gewinnung des Diarylesters umfaßt.

17. Verfahren nach Anspruch 16, worin der Monoarylester ein Glied ist aus der Gruppe bestehend aus Monophenylisophthalat und Monophenylterephthalat und das Diarylcarbonat Diphenylcarbonat ist.

18. Verfahren nach Anspruch 16, worin das molare Verhältnis des Diarylcarbonats zu dem Monoarylester 1,5 Mol bis 4 Mol des Ersteren auf 1 Mol des Letzteren beträgt.

19. Verfahren nach Anspruch 18, worin die Reaktion bei einer Temperatur von 250 bis 300°C 30 Min. bis 2 Std. lang durchgeführt wird.

## Revendications

1. Procédé de préparation des esters de diaryle d'acides aromatiques dicarboxyliques qui consiste à faire réagir un acide aromatique dicarboxylique avec un composé aromatique monohydroxylé, dans lequel l'acide est soluble et avec lequel il forme une solution, cette réaction étant effectuée en chauffant cette solution à une température élevée pour former l'ester de diaryle, et en éliminant l'eau formée pendant la réaction, caractérisé par l'utilisation d'un excès du composé aromatique monohydroxylé servant de vecteur pour éliminer l'eau, et résultant en un produit de 90—95% d'ester de diaryle avec une teneur de 5—10% de l'ester de monoaryle de l'acide dicarboxylique correspondant.

2. Procédé selon la revendication 1, dans lequel on chauffe le produit résultant avec un excès stoéchiométrique de carbonate de diaryl pour totalement convertir sa teneur en ester de monoaryl en ester de diaryl pour obtenir de l'ester de diaryle pur avec un rendement total de 99,9%.

3. Procédé selon la revendication 1, dans lequel l'acide aromatique dicarboxylique est une membre du groupe se composant de l'acide isophtalique et de l'acide téréphtalique.

4. Procédé selon la revendication 1, dans lequel l'acide aromatique dicarboxylique est l'acide isophtalique.

5. Procédé selon la revendication 1, dans lequel l'acide aromatique dicarboxylique est l'acide téréphtalique.

6. Procédé selon la revendication 1, dans lequel l'acide aromatique dicarboxylique est une membre du groupe se composant de l'acide isophtalique et de l'acide téréphtalique, et le composé aromatique monohydroxylé est le phénol.

7. Procédé selon la revendication 1, dans lequel le rapport molaire de l'acide aromatique dicarboxylique au composé aromatique monohydroxylé est 1 à 15—17, le chauffage s'effectue à 275°C à 300°C pendant 5 heures, 60—70% du composé aromatique monohydroxylé est éliminé du mélange réactionnel en même temps que l'eau formée pendant la réaction par distillation sur une période de 3—5 heures et on obtient un produit comprenant 90—95% d'ester de diaryle avec une teneur de 5—10% d'ester de monoaryle.

8. Procédé selon la revendication 1, dans lequel on utilise de l'oxyde stanneaux comme catalyseur dans la réaction.

9. Procédé selon la revendication 7, dans lequel on utilise de l'oxyde stanneaux comme catalyseur et on chauffe le produit obtenu avec un excès stoechiométrique de carbonate de diaryle pour totalement convertir l'ester de monoaryle pour obtenir de l'ester de diaryle purifiée.

10. Procédé selon la revendication 9, dans lequel l'acide aromatique dicarboxylique est un membre du groupe se composant de l'acide isophtalique et de l'acide téréphtalique; le composé aromatique monohydroxylé est le phénol, et le carbonate de diaryle est le carbonate de diphényle, et l'ester de diaryle obtenu est un membre du groupe se composant de l'isophtalate de diphényle et du téréphtalate de diphényle.

11. Procédé de purification d'un ester de diaryle d'un acide aromatique dicarboxylique brut dans lequel est présent en tant qu'impureté le demi-ester de monoaryl correspondant de cet acide, qui consiste à faire réagir ce produit brut avec un excès stoechiométrique d'un carbonate de diaryle à une température de 245°C à 300°C pendant 30 minutes à 2 heures pour totalement convertir le demi-ester en ester de diaryle, et ensuite à distiller le produit de réaction pour récupérer l'ester de diaryle de l'acide aromatique dicarboxylique pur.

12. Procédé selon la revendication 11, dans lequel l'acide aromatique dicarboxylique est un membre du groupe se composant de l'acide isophtalique et de l'acide téréphtalique, et le demi-ester de monoaryle est un membre choisi dans le groupe se composant de l'isophtalate de monophényle et du téréphtalate de monophényle.

13. Procédé selon la revendication 11 dans lequel la réaction s'effecteur en présence d'oxyde stanneaux comme catalyseur.

14. Procédé selon la revendication 12, dans lequel l'ester de diaryle est l'isophtalate de diphényle et le demi-ester de monoaryle est l'isophtalate de monophényle.

15. Procédé selon la revendication 12 dans lequel l'ester de diaryle est le téréphtalate de diphényle et le demi-ester de monoaryle est le téréphtalate de monophényle.

16. Procédé qui consiste à faire réagir un ester de monoaryle d'un acide aromatique dicarboxylique avec un excès stoéchiométrique d'un carbonate de diaryle à une température élevée pour produire l'ester de diaryle correspondant de l'acide aromatique dicarboxylique et à récupérer l'ester de diaryle.

17. Procédé selon la revendication 16, dans lequel l'ester de monoaryle est une membre du groupe se composant de l'isophtalate de monophényle et du téréphtalate de monophényle et le carbonate de diaryle est le carbonate de diphényle.

18. Procédé selon la revendication 16, dans lequel le rapport molaire du carbonate de diaryle à l'ester de monoaryle ester de 1,5 mole à 4 moles du premier pour 1 mole du dernier.

19. Procédé selon la revendication 18, dans lequel la réaction s'effecteur à une température de 250°C à 300°C et est maintenue pendant 30 minutes à 2 heures.